# EUROPEAN PATENT SPECIFICATION

(11) **EP 4 516 891 B1**
(45) Date of publication and mention of the grant of the patent: **15.07.2026**
(21) Application number: 24196048.3
(22) Date of filing: 23.08.2024
(51) Int. Cl.: C12M 3/06, C12M 1/42, C12N 15/87

(54) **METHOD, DEVICE AND SYSTEM FOR ELECTROPORATION OF BIOLOGICAL CELLS IN A FLUIDIC CHANNEL**
VERFAHREN, VORRICHTUNG UND SYSTEM ZUR ELEKTROPORATION BIOLOGISCHER ZELLEN IN EINEM FLUIDISCHEN KANAL
PROCÉDÉ, DISPOSITIF ET SYSTÈME D'ÉLECTROPORATION DE CELLULES BIOLOGIQUES DANS UN CANAL FLUIDIQUE

(30) Priority: 25.08.2023 EP 23193604
(43) Date of publication of application: 05.03.2025
(73) Proprietor: Imec VZW, 3001 Leuven (BE)
(72) Inventor: DE WIJS, Koen Johannes Hubertus Gerardus, 3010 Kessel-Lo (BE); FAUVART, Maarten, 3060 Bertem (BE); LIU, Chengxun, 3001 Heverlee (BE); STAKENBORG, Tim, 3001 Heverlee (BE)
(74) Representative: Winger

(56) References cited:
- EP-B1- 1 196 551
- US-A1- 2010 006 441
- US-A1- 2022 162 540

## Description

### Field of the Invention

The present invention relates to the field of electroporation of biological cells in a fluidic channel.

### Background of the Invention

Electroporation is a widely used technique for intracellular delivery of exogenous cargo such as DNA, RNA, proteins, and other molecules into biological cells. The technique involves applying electrical pulses to cells to temporarily permeabilize their cell membranes, allowing the cargo to enter the cells.

However, the efficiency and cell viability of electroporation are highly dependent on the characteristics of the applied electrical pulses. Pulses that are too strong or too long can cause excessive cell damage and death, while pulses that are too weak or too short may not sufficiently permeabilize the cell membrane for cargo delivery. Finding the optimal pulse parameters is challenging, as different cell types and cargo molecules have different requirements. US2022162540A1 discloses an electroporation device comprising an electroporation portion followed by a detection portion using impedance to detect the state of the cells and to control the parameters of the electric signal in the electroporation portion.

Therefore, there is still a need for methods and devices addressing at least partially the above problem.

### Summary of the Invention

It is an object of embodiments of the present invention to provide a method and device for good electroporation of biological cells flowing in a fluidic channel. This objective is accomplished by a method for electroporation of a biological cell in a fluidic channel according to the invention.

In the first aspect, the present invention relates to a method for electroporation of a biological cell in a fluidic channel, comprising steps of:
a. introducing a fluidic flow in a first direction in the fluidic channel wherein the fluidic flow comprises the cell;
b. applying a first electrical signal to the cell in the fluidic flow to produce a first electroporation at a first position in the fluidic channel;
c. measuring an impedance, i.e., EIS (electrical impedance spectroscopy), of the cell in the fluidic flow at a second position in the fluidic channel;
d. determining a second electrical signal depending on the impedance measurement of the cell in the fluidic flow at the second position in the fluidic channel;
e. applying the second electrical signal to the cell in the fluidic flow to produce a second electroporation at a third position in the fluidic channel;
wherein the first, second and third positions are sequentially arranged in the first direction of the fluidic flow.

The method advantageously customizes the second electrical signal (and the resulting field) for a second electroporation based on the impedance measurements measured, e.g., directly, after the first electroporation. Here, a solution is proposed for optimizing the electroporation settings, e.g., at single cell levels, for cells flowing in fluidic channels. Instead of a single electroporation pulse in bulk, here a cell, e.g., every cell, individually experiences at least a first electroporation pulse, followed by a second electroporation pulse, applied downstream the first pulse, that is tailored to the individual cell, based on the electroporation impact of the first electroporation pulse. To measure the impact of the first electroporation pulse, impedance cytometry is applied to the cell after the first electroporation pulse. Based on the outcome of the impedance measurements, a second pulse is applied with adjusted parameters based on the impedance measurements. By applying two pulses, the efficiency can be increased while at the same time keeping the viability high. The (e.g., immediate) impedance information after the first electroporation pulse, allows the adjustment of electroporation parameters specifically for the same cell. The adjustment of electroporation parameters can be also based on the varying characteristics within a cell population and different cell types.

In an embodiment, the impedance of the cell (e.g., at the second position) may be measured using one or a plurality of different frequencies. This allows characterizing the cell and electroporation impact in detail. For instance, it allows to extract information regarding the number of holes and size of holes after electroporation and cell characteristics such as size and internal complexity. On top of that it might be used to determine the cell position. All these extracted parameters can be used to select an optimal second electroporation pulse. The second pulse can also be omitted if the cell already has been electroporated correctly.

In an embodiment, the method may further comprise a step z. of measuring an impedance of the cell in the fluidic flow at a fourth position in the fluidic channel before applying the first electrical signal (or field) to the cell in the fluidic flow, wherein the fourth position is located before the first, second and third positions in the first direction of the fluidic flow. This provides a baseline impedance measurement of the cell before any electroporation.

In an embodiment, the method may further comprise a step y. of measuring an impedance of the cell in the fluidic flow at a fifth position in the fluidic channel after applying the second electrical signal (or field) to the cell in the fluidic flow, wherein the fifth position is located after the first, second and third positions in the first direction of the fluidic flow. This allows evaluating the impact of the second electroporation pulse.

In an embodiment, the method may further comprise sorting the cell based on the impedance measurements after the second electroporation. This enables separating successfully electroporated cells.

In an embodiment, the measurement, e.g., the impedance measurement, may be conducted in time series. This provides dynamic information on cell properties. In another embodiment, the measurement, e.g., the impedance measurement, may be conducted with a broadband frequency signal. This enables simultaneous probing at multiple frequencies. In another embodiment, the measurement, e.g., the impedance measurement, may be conducted with a selection of frequencies. This allows targeted characterization in frequency ranges of interest.

In an embodiment, the method may further comprise repeating the electroporation and impedance measurement steps. For instance, the method may further comprise repeating steps (a) to (e) or (b) to (e), or (b) to (y). In particular, repeating steps (b) to (y) after steps (b) to (y) have once been performed is particularly advantageous. This enables applying additional electroporation pulses as needed based on impedance feedback. For this purpose, the fluidic flow may be redirected upstream of the first position, or additional electroporation and measurement units may be placed downstream of the fifth position.

In an embodiment, the method may further comprise actively repositioning the cell within a transversal cross-section of the fluidic channel prior to the first electroporation, wherein the repositioning reduces the distance between the cell and the geometric center of said transversal cross-section compared to the cell's position in an upstream transversal cross-section of the fluidic channel directly before said repositioning. Centering the cell in the channel improves electroporation uniformity.

In an embodiment, the centering may be performed using at least one of fluidic focusing, acoustic focusing, or dielectrophoretic focusing. These techniques enable precise cell positioning.

In an embodiment, the fluidic flow may be controlled to maintain a constant velocity, said velocity being within a range of 0.1 mm/s to 100 mm/s. This provides sufficient residence time for electroporation while maintaining throughput.

In an embodiment, the first electrical signal (or field) and the second electrical signal (or field) may each comprise a series of electrical pulses. Multiple pulses can enhance electroporation efficiency.

In an embodiment, the electrical pulses may each have a duration between 10 microseconds and 10 milliseconds. This covers the typical range for effective electroporation.

In an embodiment, the fluidic flow may be a moving liquid comprising a cargo or the fluidic flow introduced in step a. may be a moving liquid without a cargo and the method may further comprise introducing a cargo into the fluidic channel in such a way that, during electroporation, the cell uptakes the cargo. This allows delivery of the desired cargo into the cells.

Any feature of the first aspect may be as correspondingly described in the second or third aspect.

In the second aspect, the present invention relates to an electroporation device comprising:
a fluidic channel configured for having a fluidic flow comprising a biological cell in a first direction;
a first and second electroporation units;
a first impedance unit;
wherein the first electroporation unit is configured for applying a first electrical signal (or field) to the cell in the fluidic flow at a first position in the fluidic channel so as to produce a first electroporation at the cell,
wherein the first impedance unit is configured for measuring an impedance of the cell in the fluidic flow at a second position in the fluidic channel,
wherein the second electroporation unit is configured for applying a second electrical signal (or field) to the cell in the fluidic flow at a third position in the fluidic channel so as to produce a second electroporation at the cell,
wherein the first, second and third positions are sequentially arranged in the first direction of the fluidic flow.

In other words, the device comprises a fluidic channel with a first electroporation unit, further downstream the flow, an impedance unit and after that a second electroporation unit.

In an embodiment, the fluidic channel may be a microfluidic channel.

In an embodiment, the device may further comprise a control unit configured for receiving the impedance measurement from the impedance unit and determining the second electrical signal (or field) depending on the impedance measurement from the first impedance unit. This enables adaptive control of the second electroporation based on cell-specific impedance data.

In an embodiment, any of the electroporation units, i.e., the first and/or the second electroporation units, may comprise a plurality of electrodes. Multiple electrodes allow flexible configuration of electric fields.

In an embodiment, the device may further comprise a second impedance (cytometry) unit upstream (i.e., before) of the first cell electroporation. This allows to have a reference measurement of the impedance before the electroporation. This can make the determination of the optimal second electroporation pulse more accurate and less sensitive to variations in cell position and variations within cell populations.

In an embodiment, the device may further comprise a third impedance (cytometry) unit downstream (i.e., after) of the second electroporation unit. This unit can be used to check the electroporation efficiency and cell viability. In such an embodiment, the device may further comprise a cell sorting unit configured to sort cells based on the impedance measurements of the third impedance unit. Impedance-based sorting enables enrichment of successfully electroporated cells.

In an embodiment, any of the impedance units (e.g., the first, the first and the second, the first and the third, or the first, the second and the third) may comprise a plurality of electrodes. Multiple electrodes allow flexible configuration of electric fields.

In an embodiment, any of the electroporation units (first and/or second) may comprise electrodes placed on the walls of the fluidic channel.

In an embodiment, any of the impedance units (e.g., the first, the first and the second, the first and the third, or the first, the second and the third) may comprise electrodes placed on the walls of the fluidic channel.

In an embodiment, any of the electroporation and impedance units (e.g., all such units present in the device) may have their electrodes located on a same wall or on different (e.g., opposing) walls of the fluidic channel. Same-wall electrodes simplify fabrication, making it more cost-effective, while opposite-wall electrodes provide more uniform fields. In other words, the electrodes can comprise a pair that are placed on opposing channel walls to create a uniform field or the electrodes can comprise a pair that are placed on the same side. When the pair are placed on the same side, the electrodes of the pair follow each other in the first direction, i.e., along the longitudinal direction of the channel.

To have little impact on the cell viability, behavior of the cell and prevent the formation of pores in the membrane, impedance measurements may be performed at a signal intensity which is sufficiently low to prevent the formation of pores. The changes in impedance when a flowing cell in a fluidic channel passes the electrodes are typically small. In embodiments, EIS measurements may be performed in differential mode. This is advantageous as it increases the signal to noise ratio and make it less sensitive to small changes in conductivity of the medium in the channel.

Although a reference measurement can be done at another location, it is preferably done right next to the measurement point. This way, the cell experiences two measurement points and in differential measurement this results in an S-shape in the impedance value over time.

Two configurations for the electrodes can be used: electrodes may be on opposite walls or may be coplanar.

In an embodiment, any of the impedance units may comprise 2 pairs of opposing electrodes, of which one of each pair is the reference electrode. For each pair, one electrode serves as the signal electrode, and the other serves as the reference electrode. The signal electrode has the measurement signal applied to it, which is also used as the reference signal in the lock-in amplifier. The signals are measured in differential mode to measure small differences between the signals of the electrodes. this makes the system less sensitive to small changes in conductivity of the medium and electrical signals from the surroundings.

In embodiments, for measuring the current between the electrode pair, one of the electrodes may be connected to the inputs (negative) of a differential amplifier. The differential amplifier may be considered as virtual ground since the positive input is normally connected to ground.

When the electrodes are on opposing walls, four electrodes are typically used in groups of two facing electrodes. The signal (actuation signal) is typically applied to two electrodes at the same side. The opposing electrodes are then placed at a reference value and the current is measured by measuring the voltage drop over resistances that can be placed for each actuation line, or that are placed between the two opposing electrodes and a reference value (e.g., ground). Alternatively, transimpedance amplifiers may be used to measure the current through both electrodes. The difference between the two electrical currents through the electrodes is fed into lock in amplifier.

In an embodiment, any of the impedance units may comprise 3 electrodes on the same side (i.e., following each other in the first direction on a same wall of the channel, co-planar configuration) forming two pairs by the two outer electrodes with the center electrode of which one pair is used as reference. In other words, in one embodiment, any of the impedance units may comprise three electrodes arranged in a linear sequence along the flow direction on the same wall of the channel. The center electrode acts as a shared electrode, forming two electrode pairs with the outer electrodes. One of these pairs serves as a reference for impedance measurements. A reference electrode is very advantageous because the cells are flowing in a channel typically several times larger than the cell, therefore without constricting the cells much. This makes the signal change due to a cell flowing in the channel small.

When the electrodes are coplanar, the signal (actuation signal) is typically applied to the center electrode of the three electrodes in the direction of the water flow. The outer electrodes are typically set to a predefined reference values such as ground. In embodiments, the electrical current may be measured by measuring the voltage drop over resistances that are placed between the outer electrodes and ground. In other embodiments, the electrical current may be measured by using a transimpedance amplifier that places the two outer electrodes to virtual ground and measures the current. The difference between the two electrical currents through the electrodes is typically fed into a lock-in amplifier for further processing.

In an embodiment, any of the electroporation and impedance units (e.g., all such units present in the device) may have their electrodes being pillars in the fluidic channel. In embodiments, the pillars may have at least their outer surface which is conductive. For instance, they may be made of conducting material or may be coated with conductive material. The conductive material may, for instance, be a metal or a doped semiconductor (e.g., doped silicon). Pillar electrodes enable 3D electric field shaping.

In an embodiment, any of the electroporation and impedance units (e.g., all such units present in the device) may have their electrodes being in the shape of a 3-dimensional structure in the fluidic channel. In other words, the electrodes of any of the electroporation and impedance units (e.g., all such units present in the device) may, themselves, form a 3-dimensional structure within the fluidic channel. 3D electrode configurations expand the design space. For instance, they may be shaped as pillars.

In an embodiment, any of the electroporation and impedance units (e.g., all such units present in the device) may have their electrodes embedded in a same wall or in different walls of the fluidic channel. This is advantageous as it allows the miniaturization of the device, especially in combination with a microfluidic channel, offering more control and sensitivity.

In an embodiment, the device can further comprise a cell focusing unit in the fluidic channel upstream of the first electroporation unit. In other words, in an embodiment, the device may further comprise a cell centering mechanism configured to actively reposition the cell within a transversal cross-section of the fluidic channel prior to the first electroporation, wherein the repositioning reduces the distance between the cell and the geometric center of said transversal cross-section compared to the cell's position in an upstream transversal cross-section of the fluidic channel directly before said repositioning. Centering the cell improves electroporation reproducibility. It is especially useful when the plurality of electrodes of each electroporation unit are placed on the same side of the channel, i.e., when they follow each other in the direction of the flow. Placing the electrodes on the same side can simplify fabrication but makes the position of the cell more important regarding the experienced electroporation field, which is not as uniform across the transversal cross-section of the channel compared to the situation where the electrodes face each other on opposite walls. It is also especially useful when the plurality of electrodes of each impedance unit are placed on the same side of the channel, i.e., when they follow each other in the direction of the flow.

In an embodiment, the cell centering mechanism may comprise at least one of fluidic focusing channels, acoustic transducers, or dielectrophoretic electrodes. These enable active cell positioning control.

In an embodiment, the device may further comprise a flow control unit configured to maintain the fluidic flow at a constant velocity, said velocity being within a range of 0.1 mm/s to 10 mm/s. Controlled flow enables consistent cell treatment.

In an embodiment, the device may further comprise a cargo introduction unit configured to introduce a cargo into the fluidic channel in such a way that, during electroporation, the cell uptakes the cargo. This allows automated delivery of cargo.

In the third aspect, the present invention relates to a system comprising a plurality of the electroporation devices according to any embodiments of the second aspect. For instance, the system may have a plurality of fluidic channels in parallel with each of their own units for optimal electroporation. In other words, the system may comprise multiple fluidic channels arranged in parallel, each equipped with its own set of electroporation and impedance measurement units. This enables simultaneous and individually optimized electroporation processes across multiple cells or cell populations.

In an embodiment, the system may further comprise a reservoir for storing a cargo, said reservoir being fluidically coupled to the fluidic network, said fluidic network being adapted for introducing a cargo into the fluidic channel in such a way that, during electroporation, the cell uptakes the cargo. An integrated cargo reservoir facilitates automated operation.

In an embodiment, the system may further comprise a fluidic network adapted for conducting the biological cells to the plurality of fluidic channels of the electroporation devices. A fluidic network enables parallel processing of cell samples.

In an embodiment, the system may further comprise a central control unit configured to coordinate the operation of the plurality of electroporation devices. Centralized control synchronizes the devices for optimal throughput and consistency.

It is an advantage of embodiments of the present invention that the efficiency and viability of cells can be increased by applying a second electroporation pulse that is tailored to the individual cell, based on impedance measurements taken after a first electroporation pulse. It is a further advantage of embodiments of the present invention that the second electroporation pulse can be adjusted based on the varying characteristics within a cell population and different cell types, allowing optimization of the electroporation process for a sample containing multiple cell types.

It is also an advantage of embodiments of the present invention that the cells can be sorted after electroporation based on the impedance measurements to isolate successfully electroporated cells.

Particular and preferred aspects of the invention are set out in the accompanying independent and dependent claims. Features from the dependent claims may be combined with features of the independent claims and with features of other dependent claims as appropriate and not merely as explicitly set out in the claims.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1(a) is a schematic view of an electroporation device according to embodiments of the present invention.
Fig. 1(b) is a schematic view of an electroporation device according to alternative embodiments of the present invention.
Fig. 2 is a schematic view of an electrode layout for an impedance cytometry setup according to embodiments of the present invention.

In the different figures, the same reference signs refer to the same or analogous elements.

### Detailed description of Illustrative Embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

The following terms are provided solely to aid in the understanding of the invention.

As used herein, and unless otherwise specified, the term "fluidic channel" refers to a passage, conduit, or duct through which a fluid flows. Examples of fluidic channels include microfluidic channels. A microfluidic channel is a fluidic channel with hydraulic diameter ranging from 1 to 1000 µm, preferably from 10 to 500 µm, more preferably from 15 to 350 µm, yet more preferably from 20 to 250 µm. It is typically fabricated on a chip or substrate. They are typically characterized by laminar flow due to their small diameter.

As used herein, and unless otherwise specified, the term "fluidic flow" refers to the motion or movement of a fluid through a fluidic channel.

As used herein, and unless otherwise specified, the term "first direction" refers to the primary or predominant direction in which the fluidic flow moves through the fluidic channel. The first direction is typically parallel to the longitudinal axis of the fluidic channel. Examples of the first direction include left-to-right, right-to-left, top-to-bottom, and bottom-to-top, depending on the orientation of the fluidic channel.

As used herein, and unless otherwise specified, the term "introducing a fluidic flow in a first direction" refers to the process of initiating the movement of a fluid through a fluidic channel in the first direction. Examples of methods for introducing a fluidic flow include using syringe pumps, pressure-driven flow, electrokinetic flow, or gravity-driven flow.

As used herein, and unless otherwise specified, the term "cell" refers to a biological unit that forms the basic structural and functional component of living organisms. In the context of this invention, "cell" typically denotes an eukaryotic cell, which is a membrane-bound entity containing various organelles and genetic material. Cells may be isolated from tissues, cultured cell lines, or primary cells obtained directly from organisms. Examples of cells relevant to this invention include, but are not limited to, mammalian cells such as human cell lines (e.g., HEK293, Jurkat), animal cell lines (e.g., CHO, K562), primary cells isolated from blood or tissue samples, stem cells, and genetically modified cells. The term "cells" may include cells in suspension or adherent cells that have been detached for the purpose of the described methods.

As used herein, and unless otherwise specified, the term "electrical signal" refers to a time-varying voltage field applied to the cell to induce electroporation. The electrical signal can be characterized by its amplitude, duration, frequency, and waveform. Examples of electrical signals include square pulses, exponential decay pulses, and sine waves. When the term "electrical signal" is used in any embodiment herein, it can be replaced by the term "electrical field". This is because the electrical signal on the electrodes in the fluidic channel creates an electrical field in the fluidic channel. Electroporation of cells happens by applying an electrical field over the cell. The electrical field alters the potential over the cell membrane causing nanometer sized membrane pores to form in the cell membrane.

As used herein, and unless otherwise specified, the term "determining a second electrical signal (or field) depending on the impedance measurement of the cell" refers to the process of calculating or selecting parameters for a subsequent electrical pulse or series of pulses based on the measured electrical impedance characteristics of a cell that has undergone an initial electroporation. This determination typically involves analyzing the impedance data to assess the degree of membrane permeabilization and cell viability resulting from the first electroporation. The second electrical signal is then customized to optimize the overall electroporation efficiency and maintain cell viability. Examples of parameters that may be adjusted in the second electrical signal include voltage amplitude, pulse duration, number of pulses, pulse frequency, and waveform shape. The determination may involve predefined algorithms, machine learning models, or real-time adaptive systems that interpret the impedance data and output appropriate electrical signal parameters.

As used herein, and unless otherwise specified, the term "electroporation" refers to the process of creating temporary pores or openings in the cell membrane by applying an electrical signal to the cell. Electroporation can be used to introduce various substances, such as drugs, DNA, or RNA, into the cell.

As used herein, and unless otherwise specified, the term "impedance" refers to the measure of opposition to the flow of alternating current (AC) through the cell. Impedance is typically measured by applying an AC signal to the cell and measuring the resulting voltage and current. Examples of impedance measurements include magnitude and phase angle at a single frequency or multiple frequencies.

As used herein, and unless otherwise specified, the term "cargo" refers to a substance or chemical constituent that is of interest in an analytical procedure. In the context of electroporation, a cargo is typically a substance that is introduced into the cell during the electroporation process. Examples of cargos include drugs, DNA, RNA, proteins, and dyes.

As used herein, and unless otherwise specified, the term "time series" when applied to impedance measurements refers to a sequence of impedance readings taken at multiple time points as a cell passes through a measurement region in the fluidic channel. This approach involves collecting a series of data points that represent the cell's impedance characteristics over a short period, typically milliseconds to seconds. Time series measurements can capture dynamic changes in cellular properties, providing a more comprehensive profile of the cell's electrical characteristics compared to single-point measurements. In the context of this invention, time series impedance measurements may be used to assess the effects of electroporation on cell membrane permeability, to distinguish between different cell types or states, and to inform the parameters for subsequent electroporation pulses. The time series may be obtained through multiple measurement electrodes along the channel or by taking rapid, repeated measurements as the cell passes through a single measurement region.

The invention will now be described by a detailed description of several embodiments of the invention. It is clear that other embodiments of the invention can be configured according to the knowledge of persons skilled in the art without departing from the technical teaching of the invention, the invention being limited only by the terms of the appended claims.

The present invention relates to a method for electroporation of a biological cell (1) in a fluidic channel (2), comprising the following steps:
a. introducing a fluidic flow in a first direction (4) in the fluidic channel (2), wherein the fluidic flow comprises the cell (1);
b. applying a first electrical signal (5) to the cell (1) in the fluidic flow to produce a first electroporation (6) at a first position (7) in the fluidic channel (2);
c. measuring an impedance (8) of the cell (1) in the fluidic flow at a second position (9) in the fluidic channel (2);
d. determining a second electrical signal (10) depending on the impedance measurement (8) of the cell (1) in the fluidic flow at the second position (9) in the fluidic channel (2);
e. applying the second electrical signal (10) to the cell (1) in the fluidic flow to produce a second electroporation (11) at a third position (12) in the fluidic channel (2);
wherein the first (7), second (9), and third (12) positions are sequentially arranged in the first direction (4) of the fluidic flow.

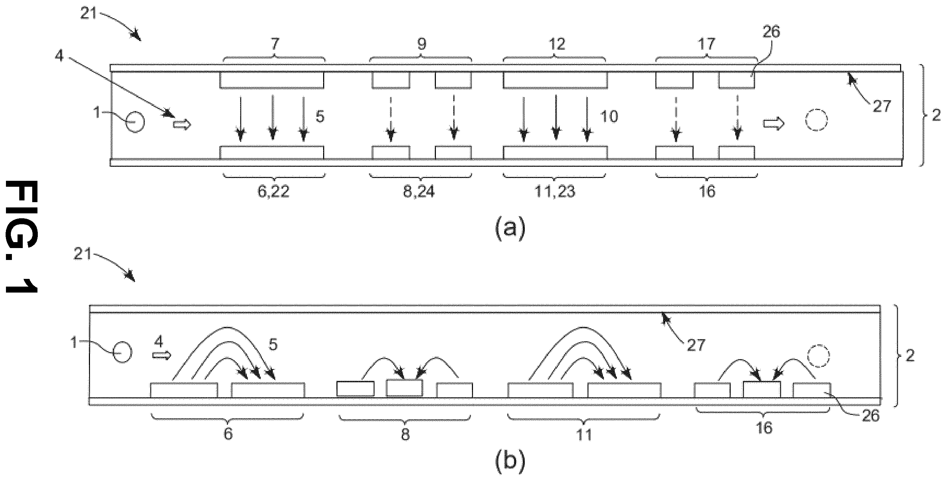

Referring to Figure 1, it illustrates a fluidic channel (2) designed for the electroporation of biological cells (1) in a continuous fluidic flow. The fluidic flow direction (4) is indicated by an arrow pointing from left to right. The channel (2) is depicted as a rectangular conduit with a series of electrodes positioned along its length.

In Figure 1(a), the fluidic channel (2) contains multiple sets of electrodes arranged sequentially. At the first position (7), a pair of electrodes applies the first electrical signal (5) to the cell (1) to produce the first electroporation (6). These electrodes are shown as rectangular blocks on opposite walls of the channel (2), creating a uniform electric field across the channel width. Following this, at the second position (9), a group of four electrodes is used for electric impedance spectroscopy (8). The third position (12) features another pair of electrodes for applying the second electrical signal (10) to produce the second electroporation (11). Finally, at the fifth position (17), another set of four electrodes is shown for a subsequent impedance measurement (16). Pairs of electrodes at each position are facing each other.

Figure 1(b) presents an alternative configuration where the electrodes are placed on the same wall (co-planar configuration) of the fluidic channel (2). The first electroporation (6) is conducted at the first position (7) using a pair of electrodes located on the bottom wall of the channel (2). The impedance measurement (8) at the second position (9) is performed using three electrodes. The center electrode is used for applying the signal, the two outer electrodes are used for measuring the impedance in differential configuration. A differential measurements scheme is applied, because it allows coping with small changes in conductivity/impedance of the medium. This can be due to change in salt concentration of the medium or changes in temperature. On top of that it suppresses electrical background signals. The impedance signals are weak, so suppressing background signals is advantageous for accurate measurements. The second electroporation (11) at the third position (12) is again conducted using a pair of electrodes on the same wall. The fifth position (17) includes another set of electrodes for impedance measurement (16). The curved arrows in this figure indicate the direction of the electric field and the flow of current between the electrodes.

Both configurations in Figures 1(a) and 1(b) demonstrate the sequential arrangement of the electroporation (6, 11) and impedance measurement (8, 16) units along the fluidic channel (2). The relative positions of the electrodes ensure that the cells (1) are processed in a controlled manner as they move through the channel (2).

As shown in Figure 1(a) and 1(b), the electroporation of the biological cell (1) is conducted at the first position (7) of the fluidic channel (2) via a pair of electrodes. The process begins with the introduction of a fluidic flow containing the cell (1), followed by the application of the first electrical signal (5) at the first position (7) to achieve the first electroporation (6).

The first electroporation (6) is conducted while the biological cell (1) is in the continuous fluidic flow. In one embodiment as shown in Figure 1(b), the electrodes for conducting the electroporation are on or adjacent to the same wall (27) of the fluidic channel (2). In another embodiment, as shown in Figure 1(a), the electrodes for conducting the electroporation are on or adjacent to the opposite walls of the fluidic channel (2).

The impedance (8) of the cell (1) is then measured at the second position (9) downstream, and based on this measurement, a second electrical signal (10) is determined and applied at the third position (12) to produce the second electroporation (11).

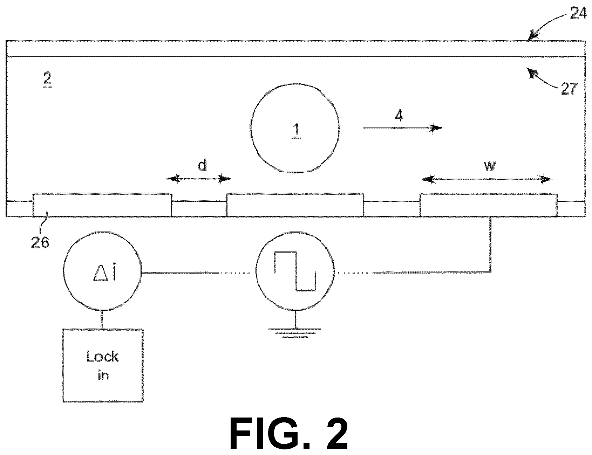

The impedance (8) of the cell (1) is measured at the second position (9) of the fluidic channel (2) in the continuous fluidic flow. In one embodiment, the impedance measurement (8) is done via a group of three electrodes where the center electrode is a reference electrode as shown in Figure 1(b) and Figure 2. The electrodes can be advantageously manufactured on or adjacent to the same wall (27) of the fluidic channel (2).

Referring to Figure 2, it illustrates a coplanar impedance cytometry setup designed for the electroporation of biological cells (1) within a fluidic channel (2). The drawing depicts a fluidic channel (2) with a biological cell (1) flowing in a first direction (4). The channel (2) is shown in a cross-sectional view, with the cell (1) represented as a circular shape moving horizontally. The setup includes a series of electrodes (26) positioned on the same wall of the fluidic channel (2). These electrodes are rectangular in shape and arranged in a linear fashion along the length of the channel (2).

The electrodes are labeled with two key dimensions: the electrode spacing (d) and the electrode width (W). The spacing (d) is the distance between adjacent electrodes, while the width (W) is the horizontal length of each electrode. The electrodes are connected to a lock-in amplifier, which processes the differential current measurement. A square wave excitation signal is applied to the electrodes, and the electrical current signals from the outer electrodes are subtracted from each other and outputted to the lock-in amplifier, which measures the impedance (8) of the cell (1) as it passes through the electric field created by the electrodes.

In another embodiment, as shown in Figure 1(a), the impedance (8) of the cell (1) is measured at the second position (9) of the fluidic channel (2) via a plurality of pairs of electrodes. The pair of electrodes can be manufactured on or adjacent to the opposite walls of the fluidic channel (2).

The second electroporation (11) is conducted after the impedance measurement (8) on the same cell (1) in the continuous fluidic flow. In one embodiment as shown in Figure 1(b), the electrodes for conducting the electroporation are on or adjacent to the same wall (27) of the fluidic channel (2). In another embodiment, as shown in Figure 1(a), the electrodes for conducting the electroporation are on or adjacent to the opposite walls of the fluidic channel (2).

In an embodiment, the width and/or height, preferably the width and the height of the fluidic channel (2) may be at least twice as large as the cell diameter. This is advantageous because it allows the cell (1) to move continuously in the fluidic flow without touching the channel wall or electrodes during electroporation and impedance measurement. It is an advantage of the present solution that the cell (1) will not get clogged in the fluidic channel (2) while being electroporated and during impedance measurement.

In embodiments, the height of each electrode of all electroporation and impedance units is at most 20%, preferably at most 10% of the height of the channel measured perpendicularly at the location of the wall where said electrode is present. This is advantageous because it allows the cell (1) to move continuously in the fluidic flow without touching the electrodes during electroporation and impedance measurement.

In an embodiment, the channel's cross-sectional area may be reduced, e.g., by at least 10%, in at least a first section of the channel where one or more of the electroporation (6, 11) and impedance (8) units are present, compared to at least a second section of the channel where no such units are present. The first and the second section may be collinear. Such a narrowing of the cross-section increases the sensitivity. The usage of larger channels reduces the chance of clogging and allows a higher throughput of cells.

In an embodiment, the impedance (8) of the cell (1) at the second position (9) may be measured using one or a plurality of different frequencies, allowing for detailed characterization of the cell (1) and the impact of electroporation.

Optionally, the device further comprises a second impedance unit (not shown in figures) at a fourth position in the fluidic channel (2) before applying the first electrical signal (5) to the cell (1) in the fluidic flow. This provides a baseline impedance measurement prior to any electroporation.

Optionally, the device further comprises a third impedance unit at a fifth position (17) in the fluidic channel (2) after applying the second electrical signal (10) to the cell (1) in the fluidic flow, wherein the fifth position (17) is located after the first (7), second (9), and third (12) positions in the first direction (4) of the fluidic flow. This allows for evaluation of the impact of the second electroporation pulse.

In one embodiment, the impedance measurement is done via a group of three electrodes where the center electrode is a reference electrode as shown in Fig. 1(b). The electrodes can be advantageously manufactured on or adjacent to the same wall (27) of the fluidic channel (2). In another embodiment, the impedance (16) of the cell (1) is measured at the second position (9) of the fluidic channel (2) via a plurality of pairs of electrodes as shown in Figure 1(a). The pair of electrodes can be manufactured on or adjacent to the opposite walls of the fluidic channel (2).

In an embodiment, the channel's cross-sectional area may be reduced, e.g., by at least 10%, in at least a first section of the channel where one or more of the electroporation (6, 11) and impedance (8) units are present, compared to at least a second section of the channel where no such units are present. The first and the second section may be collinear. Such a narrowing of the cross-section increases the sensitivity. The usage of larger channels reduces the chance of clogging and allows a higher throughput of cells.

Furthermore, the method may include measuring the impedance (16) of the cell (1) at a fifth position (17) after applying the second electrical signal (10), allowing for evaluation of the impact of the second electroporation pulse. The method may also involve sorting the cell (1) based on the impedance measurements (16) after the second electroporation, enabling the separation of successfully electroporated cells.

In an embodiment, the impedance measurement (8) may be conducted in time series, providing dynamic information on cell properties, or with a broadband frequency signal (19), allowing simultaneous probing at multiple frequencies. Alternatively, a selection of frequencies may be used for targeted characterization in specific frequency ranges.

The method may also involve repeating steps (a) to (e) or (b) to (e), or (b) to (y). In particular, repeating steps (b) to (y) after steps (b) to (y) have once been performed is particularly advantageous.

Active repositioning of the cell (1) within a transversal cross-section of the fluidic channel (2) prior to the first electroporation (6) may be performed to reduce the distance between the cell (1) and the geometric center of the transversal cross-section, improving electroporation uniformity. Focusing makes that the cells experience a more uniform signal intensity and thus will result in a more uniform electroporation. This centering may be achieved using techniques such as fluidic focusing, acoustic focusing, or dielectrophoretic focusing.

In an embodiment, the device may comprise a straight microfluidic channel with a diameter in the range of 10 to 100 µm. The microfluidic channel may be fabricated using a variety of materials and methods compatible with standard semiconductor manufacturing processes, including but not limited to CMOS technology. The channel may be constructed from polymeric materials compatible with CMOS technology, such as polydimethylsiloxane (PDMS), semiconductor materials including but not limited to silicon (Si), nitrides of semiconductor materials such as silicon nitride (SixNy), oxides of semiconductor materials such as silicon oxide (SiOx), or other passivation layer materials commonly used in semiconductor fabrication.

The choice of material allows for flexibility in the fabrication process, which may include standard CMOS techniques such as deposition, etching, and patterning. This approach is advantageous as it enables the seamless integration of various functional elements, such as electrodes, into the channel walls during the fabrication process. A significant benefit of using semiconductor materials, their oxides, nitrides, or other passivation layer materials is the compatibility with existing semiconductor manufacturing infrastructure. This compatibility facilitates the potential for large-scale production and integration with other microelectronic components.

The microfluidic channel may be fabricated using various methods, including but not limited to casting (for polymeric materials), chemical vapor deposition (CVD), physical vapor deposition (PVD), atomic layer deposition (ALD), plasma-enhanced chemical vapor deposition (PECVD), reactive ion etching (RIE), deep reactive ion etching (DRIE), and wet etching processes. The specific fabrication method may be chosen based on the selected material and the desired channel characteristics, allowing for optimization of the device's performance and functionality. In an embodiment, the bottom of the channel may be sealed with a transparent substrate such as a substrate made of glass or plexiglass. In an embodiment, the substrate may be outfitted with electrodes made of a conductive material, such as gold. In an embodiment, multiple electrode sets may be included along the channel length for electroporation and impedance measurement. In an embodiment, the electrode spacing may range from 10 to 100 µm, preferably 20 to 50 µm. In an embodiment, the electrode widths may range from 10 to 100 µm, preferably from 20 to 50 µm. In an embodiment, the layout of the electrodes may follow a coplanar impedance cytometry setup. In an embodiment, the excitation signal applied to the electrodes may be a bipolar square wave. In an embodiment, lock-in amplifiers may be used to extract specific frequency components of interest from the excitation signal. In an embodiment, the device may utilize different wavefront, e.g. square wave, sin wave, triangular wave, ... These wavefront may be used with different parameters (pulse width, pulse strength, time-frequency of the pulses)The setup allows us to perform impedance cytometry while electroporating a cell and assessing electroporation performance in fluidic flow in real time.

The description aims to develop a technique to perform electroporation and impedance cytometry. The technique will provide the basis for a dynamic electroporation optimization scheme and enable in-flow impedance monitoring to study and adjust the in-flow electroporation process.

In an embodiment, the device may comprise a straight microfluidic channel (2) with a diameter (ø) of 50 µm, cast from PDMS or other CMOS technology compatible materials such as Si or SixNy. The bottom of the channel (2) may be sealed by a (plexi)glass substrate outfitted with gold electrodes. The fluidic flow may be controlled to maintain a constant velocity within a range of 0.1 mm/s to 100 mm/s, providing sufficient residence time for electroporation while maintaining throughput. The first (5) and second (10) electrical signals may each comprise a series of electrical pulses, with pulse durations ranging from 10 microseconds to 10 milliseconds, covering the typical range for effective electroporation. The fluid flow may also comprise a cargo, or the method may further include introducing a cargo into the fluidic channel (2) to facilitate the uptake of the cargo by the cell (1) during electroporation. In an embodiment, the fluidic flow may be controlled to maintain a constant velocity within a range of 0.1 mm/s to 100 mm/s. In an embodiment, the electrical signals for electroporation may comprise a series of electrical pulses. In an embodiment, each pulse durations of the electrical signals may range from 10 microseconds to 10 milliseconds. In an embodiment, the fluid flow may comprise a cargo. In an embodiment, the method may include introducing a cargo into the fluidic channel to facilitate uptake by the cell during electroporation.

The advantage of this solution is that the efficiency and viability of the cells (1) can be increased. As there is a direct measurement of the effect of the first electroporation pulse (6), it can also help to improve the electroporation performance from a sample of multiple different cell types, in which every cell (1) requires a different electroporation pulse.

In the second aspect, the present invention relates to an electroporation device (21) comprising:
- a fluidic channel (2) configured for having a fluidic flow comprising a biological cell (1) in a first direction (4);
- a first and second electroporation unit (22, 23);
- a first impedance unit (24);
wherein the first electroporation unit (22) is configured for applying a first electrical signal (5) to the cell (1) in the fluidic flow at a first position (7) in the fluidic channel (2) to produce a first electroporation (6) at the cell (1), the first impedance unit (24) is configured for measuring an impedance (8) of the cell (1) in the fluidic flow at a second position (9) in the fluidic channel (2), and the second electroporation unit (23) is configured for applying a second electrical signal (10) to the cell (1) in the fluidic flow at a third position (12) in the fluidic channel (2) to produce a second electroporation (11) at the cell (1). The first (7), second (9), and third (12) positions are sequentially arranged in the first direction (4) of the fluidic flow.

In an embodiment, the device may further comprise a control unit configured for receiving the impedance measurement (8) from the impedance unit and determining the second electrical signal (10) based on the impedance measurement. This enables adaptive control of the second electroporation (11) based on cell-specific impedance data. The first (22) and second (23) electroporation units and/or the first impedance unit (24) may comprise a plurality of electrodes, which allow flexible configuration of electric fields. The electrodes may be located on the same wall or different walls of the fluidic channel (2), with the option of using pillar electrodes for 3D electric field shaping.

The device may also include a cell centering mechanism configured to actively reposition the cell (1) within a transversal cross-section of the fluidic channel (2) prior to the first electroporation (6), thereby improving electroporation reproducibility. This mechanism may utilize fluidic focusing channels, acoustic transducers, or dielectrophoretic electrodes for active cell positioning control. In an embodiment, the device may include structures to control the spacing between cells (1) in the fluidic flow, such as centripetal focusing in channels to initially concentrate the cells (1) before spacing them apart.

Additionally, the device may comprise a flow control unit to maintain the fluidic flow at a constant velocity within the specified range. The device may also comprise a cargo introduction unit to facilitate the uptake of cargos during electroporation. A cell sorting unit may also be included to sort cells (1) based on impedance measurements (16), enabling enrichment of successfully electroporated cells.

In the third aspect, the present invention relates to a system comprising a plurality of the electroporation devices (21) as described. The system may further include a reservoir for storing a cargo, fluidically coupled to the fluidic network, which is adapted for introducing the cargo into the fluidic channel (2) during electroporation. This integrated cargo reservoir facilitates automated operation. The system may also feature a fluidic network for conducting biological cells (1) to the plurality of fluidic channels (2) of the electroporation devices (21), enabling parallel processing of cell samples. A central control unit may coordinate the operation of the plurality of electroporation devices (21), synchronizing them for optimal throughput and consistency.

In an embodiment, the setup allows simultaneous electroporation (6, 11) and impedance cytometry by applying a bipolar square wave excitation signal typically used for electroporation to the electrodes in a coplanar arrangement (Figure 2). The relative positions of the electrodes and the cell (1) within the fluidic channel (2) are advantageous for the impedance measurement (8). The electrodes are placed such that the cell (1) moves sequentially past each electrode, allowing for continuous monitoring of the cell's impedance (8). This setup enables real-time assessment of the electroporation process by measuring changes in the cell's impedance (8) as it undergoes electroporation.

The lock-in amplifier's role is to enhance the sensitivity of the impedance measurements (8) by isolating the desired frequency components from the square wave excitation signal.

In embodiments, the electrical signals for electroporation may comprise a series of electrical pulses. In an embodiment, each pulse duration of the electrical signals may range from 10 microseconds to 10 milliseconds.

In further embodiments, the electrical signals for electroporation may have a broader range of characteristics to optimize the process for different cell types and cargo delivery requirements. The duration of individual pulses can range from 1 nanosecond to tens of milliseconds, with the strength of the electrical field varying from 0.1 kV/cm to 100 kV/cm. Various pulse strategies can be employed, including a continuous signal applied to the electrodes where cells passing in the flow stream experience a varying electrical field strength, with the duration of field application determined by the flow speed. Alternatively, a continuous unipolar or bipolar signal with a duration of 100 µs to 5 ms can be applied. Another strategy involves using triggered pulse trains, where pulses are initiated by previous impedance measurements or optical detection. These pulse trains typically consist of 2 to 5 pulses. In embodiments, the first signal of the train may be higher than the following signals. This is advantageous to create initial pores with the first pulse, while subsequent pulses increase the size of the pores. This approach allows for careful tuning to form larger pores while reducing impact on cell viability. Furthermore, at the first and second electroporation points, two different electrical signals with varying durations and intensities can be applied to optimize both cell viability and the number and size of pores formed.

### Example 1: Electroporation and Impedance Measurement of Biological Cells in a Microfluidic Channel

An experiment is being conducted to explore the electroporation of biological cells in a microfluidic channel while adapting the electroporation parameters based on real-time impedance measurements. The aim is to detect if an additional electroporation signal is needed for each cell and apply a second electroporation if required, with the goal of improving electroporation efficiency and cell viability.

A straight microfluidic channel (ø = 50 µm) is cast from PDMS and sealed with a glass substrate containing embedded gold electrodes. Multiple electrode sets for electroporation and impedance measurement are included along the channel length, with electrode spacings ranging from 20-50 µm and electrode widths of 20-50 µm. The electrode layout follows a typical coplanar impedance cytometry setup, while the excitation signal is a bipolar square wave typically used for electroporation.

Biological cells are introduced into the microfluidic channel in a continuous flow. At the first location, a pair of electrodes applies the first electroporation signal to each cell as it passes. The electrodes are either on opposite channel walls for a more uniform field or on the same wall, which simplifies fabrication but makes the cell position more critical.

Downstream, a group of three coplanar electrodes with the center electrode acting as reference is used to measure the cell impedance after the first electroporation.

Alternatively, pairs of opposing electrodes can be used. The impedance is measured at single or multiple frequencies to assess the number and size of electroporation-induced pores, as well as cell size, internal complexity, and position.

Based on the impedance data, a second electroporation signal is determined and applied further downstream via another electrode pair, unless the first electroporation is deemed sufficient. The impedance measurement provides immediate feedback on the first electroporation impact, allowing tailoring of the second electroporation pulse to each individual cell. This accounts for cell-to-cell variations and different cell types in the sample.

Optionally, an additional upstream impedance measurement provides a baseline reading before electroporation. A final downstream impedance measurement after the second electroporation allows checking the overall electroporation efficiency and cell viability.

The advantageous aspects are performing in-flow electroporation on continuously moving cells, with an impedance feedback loop between two electroporation steps to enable real-time optimization. The cell flow speed is controlled to allow sufficient time for electroporation and impedance measurements, which occur on millisecond timescales.

Cargos like DNA, RNA or proteins to be delivered into the cells are either present throughout the channel solution or introduced locally near the electroporation sites.

Focusing the cells towards the channel center, especially in the vertical direction, provides more uniform electroporation conditions.

This setup enables simultaneous electroporation and impedance cytometry on single cells, providing the basis for dynamic electroporation optimization and allowing in-flow monitoring to study and adjust the electroporation process. The dual pulse method with impedance feedback aims to increase electroporation efficiency and cell viability compared to a single pulse.

### Example 2: Electroporation with Multiple Pulse Sequences

An experiment is being conducted to explore the effects of applying multiple electroporation pulse sequences to biological cells in a microfluidic channel. The aim is to investigate if repeated electroporation pulses, with impedance measurements between each sequence, can further improve transfection efficiency and cell viability.

A microfluidic device is fabricated with a main channel (width = 100 µm, height = 50 µm) containing multiple electroporation and impedance measurement regions. The device includes an initial impedance measurement region, followed by a first electroporation region, then a second impedance measurement region, a second electroporation region, a third impedance measurement region, a third electroporation region, and finally a final impedance measurement region.

Each electroporation region consists of gold electrodes (width = 30 µm, spacing = 40 µm) on opposite channel walls. Impedance measurement regions use three coplanar electrodes (width = 20 µm, spacing = 30 µm) on the channel bottom. HEK293 cells suspended in electroporation buffer containing GFP-encoding plasmids are introduced into the channel at a flow rate of 100 µL/hr. The initial impedance measurement provides a baseline reading for each cell.

At the first electroporation region, cells receive a pulse sequence (5 pulses, 3 ms duration, 1 Hz frequency) with voltage amplitude determined based on cell size from the initial impedance reading. Immediately downstream, the second impedance measurement assesses the impact of the first electroporation.

Based on this measurement, the second electroporation pulse sequence is adjusted.
If the first electroporation appears insufficient, a stronger pulse sequence is applied. If it appears optimal or excessive, a weaker sequence or no additional pulses are used. This process is repeated for the third electroporation region, with pulse parameters fine-tuned based on the cumulative effects observed from the impedance measurements.

The final impedance measurement provides data on the overall changes to the cell membrane permeability and viability after the complete electroporation process.

Cells are collected at the outlet and cultured for 48 hours before analyzing GFP expression via flow cytometry to assess transfection efficiency. Cell viability is determined using a live/dead staining assay.

This multi-pulse, feedback-controlled approach allows for gentler initial electroporation followed by targeted additional pulses only when necessary. The experiment aims to achieve higher transfection rates and improved viability compared to single high-intensity pulse methods by tailoring the electroporation process to each individual cell's response.

### Example 3: Electroporation with Localized Cargo Delivery

An experiment is being designed to investigate the effectiveness of localized cargo delivery during in-flow electroporation in a microfluidic device. The goal is to minimize cargo consumption and improve delivery efficiency by introducing the cargo molecules only at the electroporation sites. This approach offers several advantages, including reduced cargo consumption, lower chance of spontaneous cargo integration into cells, and decreased cargo degradation due to minimal exposure time.

A PDMS microfluidic device is fabricated with a main channel (width = 80 µm, height = 40 µm) for cell flow and two side channels for localized cargo introduction. The device layout includes a cell focusing region using inertial microfluidics, followed by a first impedance measurement region, then a first electroporation and cargo delivery region, a second impedance measurement region, a second electroporation and cargo delivery region, and finally a final impedance measurement region.

The electroporation regions consist of gold electrodes (width = 25 µm, spacing = 35 µm) on opposite channel walls. Immediately upstream of each electroporation region, a side channel (width = 20 µm) intersects the main channel at a 45° angle.
Jurkat cells suspended in electroporation buffer are introduced into the main channel at 50 µL/hr. The cell focusing region uses asymmetric curves to position cells near the channel center. The initial impedance measurement provides baseline cell data.

A fluorescently-labeled siRNA solution is introduced via the side channels at 5 µL/hr using precision syringe pumps. This method creates a localized high concentration of cargo at each electroporation site by forming a thin stream of cargo solution that merges with the main cell flow just before the electrodes. The 45° angle of the side channels helps to minimize disruption to the main flow while ensuring efficient mixing.

The flow rates are carefully optimized through computational fluid dynamics simulations and experimental validation to ensure the cargo stream envelops the cells without significant dilution before reaching the electroporation region. This precise control over cargo introduction significantly reduces the total amount of cargo needed compared to traditional bulk electroporation methods, where the entire cell solution contains the cargo molecules.At the first electroporation site, cells receive a pulse sequence (3 pulses, 2 ms duration, 2 kHz frequency) with voltage determined based on the initial impedance reading. The second impedance measurement assesses the first electroporation's impact.

The second electroporation parameters are adjusted based on this measurement. If the first electroporation appears insufficient, a stronger pulse sequence is applied. If optimal or excessive, a weaker sequence or no additional pulses are used.

The final impedance measurement provides data on overall changes to cell membrane permeability and viability after the complete process.

Cells are collected at the outlet and cultured for 24 hours before analyzing siRNA uptake via flow cytometry to assess delivery efficiency. Cell viability is determined using a live/dead staining assay.

This localized delivery approach aims to significantly reduce cargo consumption compared to methods where the entire cell solution contains the cargo molecules. By introducing the cargo only at the electroporation sites and using impedance feedback to optimize pulse parameters, the experiment seeks to achieve efficient and targeted delivery while maintaining high cell viability.

### Example 4: Electroporation with Acoustic Cell Focusing and Sorting

This experiment is designed to integrate acoustic cell focusing and sorting capabilities with the adaptive electroporation system. The goal is to improve electroporation uniformity by precisely positioning cells prior to electroporation and to sort cells based on the outcome of the process.
A silicon-glass microfluidic device is fabricated with a main channel (width = 375 µm, height = 150 µm) and several key components. These include an acoustic focusing region, an initial impedance measurement region, a first electroporation region, a second impedance measurement region, a second electroporation region, a final impedance measurement region, and an acoustic sorting region.
The acoustic focusing region uses a piezoelectric transducer (resonance frequency = 2 MHz) to create a standing wave field, aligning cells along the channel center. This ensures consistent cell positioning for subsequent operations. Electroporation regions consist of 3D electrodes (height = 100 µm, width = 50 µm, spacing = 75 µm) extending from the channel floor and ceiling. This design provides a more uniform electric field compared to co-planar electrodes.
CHO cells suspended in electroporation buffer containing GFP-encoding mRNA are introduced into the channel at 200 µL/hr. After acoustic focusing, the initial impedance measurement provides baseline cell data.
At the first electroporation site, three different pulsing strategies are implemented and compared:
1. Continuous Pulsing (Option 1): In this approach, a continuous pulse sequence (4 pulses, 1.5 ms duration, repeated every 10 ms) is applied at the electroporation site without taking into account the real-time location of cells. The voltage is determined based on the initial impedance reading. This method ensures that all cells passing through the region receive pulses but may result in some cells receiving multiple pulse trains or partial exposure.
2. Impedance-Triggered Pulsing (Option 2): The device is configured to perform rapid impedance measurements just before the electroporation region. When a cell is detected through a change in impedance, a single pulse sequence (4 pulses, 1.5 ms duration) is triggered. The voltage is determined based on the initial impedance reading and adjusted according to the pre-electroporation impedance measurement. This method aims to apply pulses more precisely when cells are in the optimal position.
3. Optical Sensing-Triggered Pulsing (Option 3): The device uses real-time optical sensing (e.g., high-speed imaging or integrated optical sensors) to detect cells entering the electroporation region. When a cell is detected, a pulse sequence (4 pulses, 1.5 ms duration) is triggered. The voltage is determined based on the initial impedance reading. This method offers the highest precision in terms of pulse timing relative to cell position. The second impedance measurement assesses the first electroporation's impact.
   For the second electroporation site, the pulsing strategy is adjusted based on the results from the first site:
   - If using Option 1 (Continuous Pulsing): The pulse parameters (voltage, duration, or frequency) are adjusted based on the second impedance measurement. The continuous pulsing continues with these new parameters.
   - If using Option 2 (Impedance-Triggered Pulsing): The system continues to use impedance measurements to trigger pulses, but the pulse parameters are adjusted based on the second impedance measurement. If the first electroporation appears insufficient, a stronger pulse sequence is applied. If optimal or excessive, a weaker sequence or no additional pulses are used.
   - If using Option 3 (Optical Sensing-Triggered Pulsing): The system continues to use optical sensing to trigger pulses, with pulse parameters adjusted based on the second impedance measurement. This allows for precise timing and adaptive pulsing for each individual cell. The final impedance measurement provides data on overall changes to cell membrane permeability and viability after the complete process.

Based on the final impedance measurement, cells are sorted using a microfluidic sorting region. Acoustic actuators at the junction where the main channel splits into two outlet channels. The acoustic actuator can deflect cells based on their measured impedance values after electroporation into one of two outlets: successfully electroporated cells (optimal impedance change), and others. The acoustic sorter can be replaced with a bubble-jet actuator. These bubble-jet actuators generate rapid, localized bubble formation through brief heating pulses. The bubbles create transient fluid jets that can deflect. This bubble jet sorting method allows for rapid, on-demand cell sorting. Cells from each outlet are collected and cultured for 12 hours before analyzing GFP expression via flow cytometry to assess transfection efficiency. Cell viability is determined using a live/dead staining assay.

This integrated approach aims to improve electroporation uniformity through precise cell positioning, optimize the process for individual cells through adaptive pulsing, and increase overall efficiency by sorting cells based on electroporation outcome. The combination of acoustic focusing manipulation with impedance-based feedback and sorting offers a comprehensive solution for high-throughput, optimized cell electroporation.

### Example 5: Electroporation with Dielectrophoretic Cell Positioning and Multiple Cargo Delivery

This experiment is designed to explore the use of dielectrophoresis (DEP) for precise cell positioning prior to electroporation, combined with the capability to deliver multiple distinct cargos to different cell populations. The goal is to achieve highly controlled electroporation conditions and enable multiplexed cargo delivery in a single device.

A glass microfluidic device is fabricated with a main channel (width = 200 µm, height = 80 µm) and several key components. These include a DEP focusing region, an initial impedance measurement region, a first electroporation region with cargo delivery, a second impedance measurement region, a second electroporation region with cargo delivery, a final impedance measurement region, and a DEP-based sorting region.

The DEP focusing region uses interdigitated electrodes on the channel floor (electrode width = 20 µm, spacing = 20 µm) to create non-uniform electric fields. By applying an AC signal (10 Vpp, 5 MHz), cells are aligned along the channel centerline due to negative DEP forces.

Electroporation regions consist of 3D electrodes (height = 60 µm, width = 40 µm, spacing = 60 µm) extending from the channel floor and ceiling. Immediately upstream of each electroporation region, two side channels (width = 30 µm) intersect the main channel at 30° angles on opposite sides.

A mixed population of two different cells suspended in low-conductivity electroporation buffer is introduced into the channel at 150 µL/hr. After DEP focusing, the initial impedance measurement provides baseline cell data and allows for cell type discrimination based on their dielectric properties.

Two different fluorescently-labeled RNA or DNA solutions are introduced via the side channels at 10 µL/hr each. The flow rates are optimized to ensure the cargo streams envelop the cells without significant mixing before reaching the electroporation region.

At the first electroporation site, cells receive a pulse sequence (3 pulses, 2 ms duration, 2 Hz frequency) with voltage determined based on the initial impedance reading and identified cell type. The second impedance measurement assesses the first electroporation's impact.

The second electroporation parameters are adjusted based on this measurement. If the first electroporation appears insufficient, a stronger pulse sequence is applied. If optimal or excessive, a weaker sequence or no additional pulses are used.

The final impedance measurement provides data on overall changes to cell membrane permeability and viability after the complete process.

Based on the final measurement and initial cell type identification, cells are sorted using a DEP-based sorting region. Interdigitated electrodes with a chevron pattern are used to deflect cells into one of three outlet channels: successfully electroporated a first type of cells, successfully electroporated a second type of cells, and under-electroporated or potentially damaged cells.

Cells from each outlet are collected and cultured for 18 hours before analyzing GFP and RFP expression via flow cytometry to assess transfection efficiency and specificity. Cell viability is determined using a live/dead staining assay.

This integrated approach aims to achieve precise control over cell positioning and electroporation conditions, enable cell type-specific cargo delivery, and sort cells based on both their type and electroporation outcome. The combination of DEP manipulation, impedance-based feedback, and multiplexed cargo delivery offers a sophisticated platform for high-throughput, cell type-specific electroporation and transfection.

It is to be understood that although preferred embodiments, specific constructions and configurations, as well as materials, have been discussed herein for devices according to the present invention, various changes or modifications in form and detail may be made without departing from the scope of this invention. For example, any formulas given above are merely representative of procedures that may be used. Functionality may be added or deleted from the block diagrams and operations may be interchanged among functional blocks. Steps may be added or deleted to methods described within the scope of the present invention.

## Claims

1. A method for electroporation of a biological cell (1) in a fluidic channel (2), comprising steps of:
a. introducing a fluidic flow in a first direction (4) in the fluidic channel (2) wherein the fluidic flow comprises the cell (1);
b. applying a first electrical signal (5) to the cell (1) in the fluidic flow to produce a first electroporation (6) at a first position (7) in the fluidic channel (2);
c. measuring an impedance (8) of the cell (1) in the fluidic flow at a second position (9) in the fluidic channel (2);
d. determining a second electrical signal (10) depending on the impedance measurement (8) of the cell (1) in the fluidic flow at the second position (9) in the fluidic channel (2);
e. applying the second electrical signal (10) to the cell (1) in the fluidic flow to produce a second electroporation (11) at a third position (12) in the fluidic channel (2);
wherein the first (7), second (9) and third (12) positions are sequentially arranged in the first direction (4) of the fluidic flow.

2. The method according to claim 1, wherein the impedance (8) of the cell (1) at the second position (9) is measured using one or a plurality of different frequencies.

3. The method according to any of the preceding claims, further comprising a step of:
z. measuring an impedance of the cell (1) in the fluidic flow at a fourth position in the fluidic channel (2) before applying the first electrical signal (5) to the cell (1) in the fluidic flow, wherein the fourth position is located before the first (7), second (9) and third (12) positions in the first direction (4) of the fluidic flow.

4. The method according to any of the preceding claims, further comprising a step of:
y. measuring an impedance (16) of the cell (1) in the fluidic flow at a fifth position (17) in the fluidic channel (2) after applying the second electrical signal (10) to the cell (1) in the fluidic flow, wherein the fifth position (17) is located after the first (7), second (9) and third (12) positions in the first direction (4) of the fluidic flow.

5. The method according to any of the preceding claims, wherein the impedance measurement (8) is conducted in time series.

6. The method according to any of the preceding claims, wherein the impedance measurement (8) is conducted with a selection of frequencies.

7. The method according to any of the preceding claims, further comprising repeating steps (a) to (e).

8. An electroporation device (21) comprising:
a fluidic channel (2) configured for having a fluidic flow comprising a biological cell (1) in a first direction (4);
a first (22) and second (23) electroporation units;
a first impedance unit (24);
wherein the first electroporation unit (22) is configured for applying a first electrical signal (5) to the cell (1) in the fluidic flow at a first position (7) in the fluidic channel (2) so as to produce a first electroporation (6) at the cell (1),
wherein the first impedance unit (24) is configured for measuring an impedance (8) of the cell (1) in the fluidic flow at a second position (9) in the fluidic channel (2),
wherein the second electroporation unit (23) is configured for applying a second electrical signal (10) to the cell (1) in the fluidic flow at a third position (12) in the fluidic channel (2) so as to produce a second electroporation (11) at the cell (1),
wherein the first (7), second (9) and third (12) positions are sequentially arranged in the first direction (4) of the fluidic flow.

9. The electroporation device (21) according to claim 8, further comprising a control unit configured for receiving the impedance measurement (8) from the impedance unit (24) and determining the second electrical signal (10) depending on the impedance measurement (8) from the first impedance unit (24).

10. The electroporation device (21) according to any of claims 8 and 9, wherein the first (22) and second (23) electroporation units and/or the first impedance unit (24) comprises a plurality of electrodes (26).

11. The electroporation device (21) according to claim 10, wherein the electrodes (26) are located on a same wall (27) or different walls of the fluidic channel (2).

12. The electroporation device (21) according to any of claims 10 or 11, wherein the electrodes (26) are pillars in the fluidic channel (2).

13. The electroporation device (21) according to any of claims 10-12, wherein the electrodes (26) themselves form a 3-dimensional structure in the fluidic channel (2).

14. A system comprising a plurality of the electroporation devices (21) according to any of claims 8 to 13.

## Patentansprüche

1. Ein Verfahren zur Elektroporation einer biologischen Zelle (1) in einem fluidischen Kanal (2), umfassend die folgenden Schritte:
a.- Einbringen eines fluidischen Stroms in einer ersten Richtung (4) in den fluidischen Kanal (2), wobei der fluidischen Strom die Zelle (1) umfasst;
b.- Anlegen eines ersten elektrischen Signals (5) an die Zelle (1) in dem fluidischen Strom, um eine erste Elektroporation (6) an einer ersten Position (7) in dem fluidischen Kanal (2) zu erzeugen;
c.- Messen einer Impedanz (8) der Zelle (1) in dem fluidischen Strom an einer zweiten Position (9) in dem fluidischen Kanal (2);
d.- Bestimmen eines zweiten elektrischen Signals (10) in Abhängigkeit von der Impedanzmessung (8) der Zelle (1) in dem fluidischen Strom an der zweiten Position (9) in dem fluidischen Kanal (2);
e.- Anlegen des zweiten elektrischen Signals (10) an die Zelle (1) in dem fluidischen Strom, um eine zweite Elektroporation (11) an einer dritten Position (12) in dem fluidischen Kanal (2) zu erzeugen;
wobei die erste (7), zweite (9) und dritte (12) Position nacheinander in der ersten Richtung (4) des fluidischen Stroms angeordnet sind.

2. Das Verfahren nach Anspruch 1, wobei die Impedanz (8) der Zelle (1) an der zweiten Position (9) mit einer oder einer Vielzahl verschiedenen Frequenzen gemessen wird.

3. Das Verfahren nach einem der vorstehenden Ansprüche, ferner umfassend einen Schritt zum:
z. Messen einer Impedanz der Zelle (1) in dem fluidischen Strom an einer vierten Position in dem fluidischen Kanal (2), bevor das erste elektrische Signal (5) an die Zelle (1) in dem fluidischen Strom angelegt wird, wobei sich die vierte Position vor der ersten (7), zweiten (9) und dritten (12) Position in der ersten Richtung (4) des fluidischen Stroms befindet.

4. Das Verfahren nach einem der vorstehenden Ansprüche, ferner umfassend einen Schritt zum:
y. Messen der Impedanz (16) der Zelle (1) in dem fluidischen Strom an einer fünften Position (17) in dem fluidischen Kanal (2) nachdem das zweite elektrische Signal (10) an die Zelle (1) in dem fluidischen Strom angelegt wird, wobei sich die fünfte Position (17) nach der ersten (7), zweiten (9) und dritten (12) Position in der ersten Richtung (4) des fluidischen Stroms befindet.

5. Das Verfahren nach einem der vorstehenden Ansprüche, wobei die Impedanzmessung (8) in Zeitreihen durchgeführt wird.

6. Das Verfahren nach einem der vorstehenden Ansprüche, wobei die Impedanzmessung (8) mit einer Auswahl von Frequenzen durchgeführt wird.

7. Das Verfahren nach einem der vorstehenden Ansprüche, ferner umfassend Wiederholen von Schritten (a) bis (e).

8. Eine Elektroporationsvorrichtung (21) umfassend:
einen fluidischen Kanal (2), der so konfiguriert ist, dass er einen fluidischen Strom, der eine biologische Zelle (1) umfasst, in einer ersten Richtung (4) aufweist;
eine erste (22) und eine zweite (23) Elektroporationseinheit;
eine erste Impedanzeinheit (24);
wobei die erste Elektroporationseinheit (22) so konfiguriert ist, dass sie an einer ersten Position (7) in dem fluidischen Kanal (2) in dem fluidischen Strom ein erstes elektrisches Signal (5) an die Zelle (1) anlegt, um eine erste Elektroporation (6) an der Zelle (1) zu erzeugen.
wobei die erste Impedanzeinheit (24) so konfiguriert ist, dass sie eine Impedanz (8) der Zelle (1) in dem fluidischen Strom an einer zweiten Position (9) in dem fluidischen Kanal (2) misst,
wobei die zweite Elektroporationseinheit (23) so konfiguriert ist, dass sie an einer dritten Position (12) in dem fluidischen Kanal (2) in dem fluidischen Strom ein zweites elektrisches Signal (10) an die Zelle (1) anlegt, um eine zweite Elektroporation (11) an der Zelle (1) zu erzeugen,
wobei die erste (7), zweite (9) und dritte (12) Position nacheinander in der ersten Richtung (4) des fluidischen Stroms angeordnet sind.

9. Die Elektroporationsvorrichtung (21) nach Anspruch 8, ferner umfassend eine Steuereinheit, die so konfiguriert ist, dass sie die Impedanzmessung (8) von der Impedanzeinheit (24) empfängt und das zweite elektrische Signal (10) in Abhängigkeit von der Impedanzmessung (8) von der ersten Impedanzeinheit (24) bestimmt.

10. Die Elektroporationsvorrichtung (21) nach einem der Ansprüche 8 und 9, wobei die erste (22) und die zweite (23) Elektroporationseinheit und/oder die erste Impedanzeinheit (24) eine Vielzahl von Elektroden (26) umfassen.

11. Die Elektroporationsvorrichtung (21) nach Anspruch 10, wobei sich die Elektroden (26) an einer selben Wand (27) oder an verschiedenen Wänden des fluidischen Kanals (2) befinden.

12. Die Elektroporationsvorrichtung (21) nach einem der Ansprüche 10 oder 11, wobei die Elektroden (26) Säulen in dem fluidischen Kanal (2) sind.

13. Die Elektroporationsvorrichtung (21) nach einem der Ansprüche 10-12, wobei die Elektroden (26) selbst eine dreidimensionale Struktur in dem fluidischen Kanal (2) bilden.

14. Ein System, umfassend eine Vielzahl der Elektroporationsvorrichtungen (21) nach einem der Ansprüche 8 bis 13.

## Revendications

1. Un procédé d'électroporation d'une cellule biologique (1) dans un canal fluidique (2), comprenant les étapes de :
a. introduire un flux fluidique dans une première direction (4) dans le canal fluidique (2) dans lequel le flux fluidique comprend la cellule (1) ;
b. appliquer un premier signal électrique (5) à la cellule (1) dans le flux fluidique pour produire une première électroporation (6) à une première position (7) dans le canal fluidique (2) ;
c. mesurer une impédance (8) de la cellule (1) dans le flux fluidique à une deuxième position (9) dans le canal fluidique (2) ;
d. déterminer un deuxième signal électrique (10) en fonction de la mesure d'impédance (8) de la cellule (1) dans le flux fluidique à la deuxième position (9) dans le canal fluidique (2) ;
e. appliquer le deuxième signal électrique (10) à la cellule (1) dans le flux fluidique pour produire une deuxième électroporation (11) à une troisième position (12) dans le canal fluidique (2) ;
dans lequel les première (7), deuxième (9) et troisième (12) positions sont disposées séquentiellement dans la première direction (4) du flux fluidique.

2. Le procédé selon la revendication 1, dans lequel l'impédance (8) de la cellule (1) à la deuxième position (9) est mesurée en utilisant une ou plusieurs fréquences différentes.

3. Le procédé selon l'une quelconque des revendications précédentes, comprenant en outre une étape de :
z. mesurer une impédance de la cellule (1) dans le flux fluidique à une quatrième position dans le canal fluidique (2) avant d'appliquer le premier signal électrique (5) à la cellule (1) dans le flux fluidique, dans lequel la quatrième position est située avant les première (7), deuxième (9) et troisième (12) positions dans la première direction (4) du flux fluidique.

4. Le procédé selon l'une quelconque des revendications précédentes, comprenant en outre une étape de :
y. mesurer une impédance (16) de la cellule (1) dans le flux fluidique à une cinquième position (17) dans le canal fluidique (2) après avoir appliqué le deuxième signal électrique (10) à la cellule (1) dans le flux fluidique, dans lequel la cinquième position (17) est située après les première (7), deuxième (9) et troisième (12) positions dans la première direction (4) du flux fluidique.

5. Le procédé selon l'une quelconque des revendications précédentes, dans lequel la mesure d'impédance (8) est effectuée en série temporelle.

6. Le procédé selon l'une quelconque des revendications précédentes, dans lequel la mesure d'impédance (8) est effectuée avec une sélection de fréquences.

7. Le procédé selon l'une quelconque des revendications précédentes, comprenant en outre la répétition des étapes (a) à (e).

8. Un dispositif d'électroporation (21) comprenant :
un canal fluidique (2) configuré pour avoir un flux fluidique comprenant une cellule biologique (1) dans une première direction (4) ;
une première (22) et une deuxième (23) unités d'électroporation ;
une première unité d'impédance (24) ;
dans lequel la première unité d'électroporation (22) est configurée pour appliquer un premier signal électrique (5) à la cellule (1) dans le flux fluidique à une première position (7) dans le canal fluidique (2) afin de produire une première électroporation (6) à la cellule (1),
dans lequel la première unité d'impédance (24) est configurée pour mesurer une impédance (8) de la cellule (1) dans le flux fluidique à une deuxième position (9) dans le canal fluidique (2),
dans lequel la deuxième unité d'électroporation (23) est configurée pour appliquer un deuxième signal électrique (10) à la cellule (1) dans le flux fluidique à une troisième position (12) dans le canal fluidique (2) afin de produire une deuxième électroporation (11) à la cellule (1),
dans lequel les première (7), deuxième (9) et troisième (12) positions sont disposées séquentiellement dans la première direction (4) du flux fluidique.

9. Le dispositif d'électroporation (21) selon la revendication 8, comprenant en outre une unité de contrôle configurée pour recevoir la mesure d'impédance (8) de l'unité d'impédance (24) et déterminer le deuxième signal électrique (10) en fonction de la mesure d'impédance (8) de la première unité d'impédance (24).

10. Le dispositif d'électroporation (21) selon l'une quelconque des revendications 8 et 9, dans lequel les première (22) et deuxième (23) unités d'électroporation et/ou la première unité d'impédance (24) comprennent une pluralité d'électrodes (26).

11. Le dispositif d'électroporation (21) selon la revendication 10, dans lequel les électrodes (26) sont situées sur une même paroi (27) ou sur des parois différentes du canal fluidique (2).

12. Le dispositif d'électroporation (21) selon l'une quelconque des revendications 10 ou 11, dans lequel les électrodes (26) sont des piliers dans le canal fluidique (2).

13. Le dispositif d'électroporation (21) selon l'une quelconque des revendications 10 à 12, dans lequel les électrodes (26) forment elles-mêmes une structure tridimensionnelle dans le canal fluidique (2).

14. Un système comprenant une pluralité des dispositifs d'électroporation (21) selon l'une quelconque des revendications 8 à 13.
